# EUROPEAN PATENT SPECIFICATION

(11) **EP 2 644 202 B1**
(45) Date of publication and mention of the grant of the patent: **13.01.2016**
(21) Application number: 13001347.7
(22) Date of filing: 15.03.2013
(51) Int. Cl.: A61K 36/14, A61P 17/06, A61P 17/02

(54) **Ointment for skin treatment, and method of its manufacture**
Salbe zur Hautbehandlung, und Herstellungsverfahren dafür
Onguent pour le traitement de la peau et son procédé de fabrication

(30) Priority: 27.03.2012 ME 201240
(43) Date of publication of application: 02.10.2013
(73) Proprietor: Nurkovic, Suljo, 84310 Rozaje (ME)
(72) Inventor: Nurkovic, Suljo, 84310 Rozaje (ME)
(74) Representative: Diehl & Partner GbR

(56) References cited:
- WO-A1-2005/063266
- DATABASE WPI Week 200975 Thomson Scientific, London, GB; AN 2009-P88235 XP002703213, & KR 2009 0103000 A (HOPKINS BIO RES CENT) 1 October 2009 (2009-10-01)
- TUMEN I ET AL: "A therapeutic approach for wound healing by using essential oils of cupressus and Juniperus species growing in Turkey", EVIDENCE-BASED COMPLEMENTARY AND ALTERNATIVE MEDICINE 2012 HINDAWI PUBLISHING CORPORATION USA, vol. 2012, 2012, XP009171225, ISSN: 1741-427X

## Description

The present invention relates to an ointment effective in the treatment of the skin, such as burns and psoriasis.

The treatment of wounds and other damages to the epidermis is conceivably the oldest medicinal activity of mankind. Nevertheless, so far no broadly effective treatment has been found. Some proposals have been made on the basis of herbal extracts (one example is EP 0 967 988 B1), but have proven to be of limited potential. Other proposals based on synthetic medicaments have adverse side effects, some of which may be severe. Thus, in recent years, the demand for a purely natural medicament has increased.

The article "A Therapeutic Approach for Wound Healing by Using Essential Oils of Cupressus and Juniperus Species Growing in Turkey" by Tumen et al., XP009171225, discloses essential oils obtained from cones of *Cupressus* and berries from *Juniperus* which were evaluated for their wound-healing and anti-inflammatory effects. The essential oils of *J. oxycedrus* subsp. *oxycedrus* and *J. phoenicea* demonstrated the highest activities, while the rest of the species did not show any significant wound healing effect.

The present invention aims at overcoming the drawbacks of the prior art, and at providing an ointment that has a broader scope of indications and/or is more effective.

In this regard, the invention provides an ointment for use in a therapeutic method of topical treatment of skin lesions according to claim 1, namely an ointment comprising an extract of juniper berries, *Juniperus comm. fruct.,* and at least one extract selected from *Symphytum off., Calendula off., Plantago lanceolata* and *Matricaria chamomilla* in a fatty base; a method of manufacturing the ointment according to claim 8; and a composition for use in the topical treatment of lesions of the skin according to claim 15.

In the present application, treatment is to be understood as topical treatment. The extracts are made by admixing the ingredients to a fatty base, prolonged heating and subsequent filtering, and are therefore considered to be lipophilic; a detailed chemical analysis is not available.

The inventor has found that the ointment is effective in the treatment of burns including sunburns, herpes-induced lesions, neurodermitis, psoriasis, acne, insect bites or stings, hematoms, bacterially or virally infected lesions of the skin and mucous membranes, and fungal lesions.

According to a first example, 832 g of suet were mixed with 125 g each of juniper berries *(Juniperus comm. fruct.),* roots of *Symphytum off.,* flowers of *Calendula off.,* leaves of *Plantago lanceolata,* and flowers of *Matricaria chamomilla,* each of these ingredients in macerated dried form. These ingredients form a first group. The proportion by weight was thus 8.6 % for each of the five ingredients and 57 % for the suet as the fatty base. In general, the weight proportion of each of the ingredients may be 6.5 % to 9.5 %, or more generally 5-10 %. Accordingly, the percentage by weight of the fatty base may range from 50 % to 60 % or even to 65 %.

According to a second example, the final ointment contains an additional 40 g of beeswax, or more generally 30-100 g corresponding to 2 - 3.5 % or -7 % by weight of the mixture.

In either case, the suet is heated to 150°C-200°C and held at this temperature for 5 minutes. The ingredients are then added to the heated fat and left to simmer for about an hour. Thereafter, the pot is left to cool for about 24 hrs. Then, the mixture is heated again to become just flowable (about 30-40°C), and filtered by pressing it through a silk cloth. In the second example, the beeswax is then added and the mixture is again heated (to about 60-100°C) and stirred to homogenize. In either case, the filtrate is then cooled and stored in the cool.

The constituents of *Juniperus comm.,* α-pinene, sabinene, α-cadinene, α-terpinene, γ-terpinene, and α-tujene (about 2.5 % ethereal oils), bring about the meritorious effect in combination with other ingredients of the first group. Juniper berries further contain invert sugar (33 %), resins (10 %), tannins (8 %), waxes, flavonoids, and juniperozide.

According to embodiments, ingredients of a second group are further added, namely *Equiseti herba* and/or *Hypericum perforatum.* Other constituents are not added, however.

The ointment is suitable for the treatment of skin and mucous skin (such as of nose, mouth, genital, and anal). In particular, the ointment is substantially free of heavy metals, such as lead, cadmium, chromium, nickel, arsen, and mercury. The ointment is usually slightly acidic (pH 4-5).

The fatty base is mainly (>50 % by weight) suet, i.e. bovine fat. If this is not available, a plant oil such as olive oil may additionally or alternatively be used.

It may be noted that while not all ingredients (except for juniper berries) are strictly required, the best results are usually obtained with the extracts of the first group in combination with juniper.

## Claims

1. An ointment for use in a therapeutic method of topical treatment of skin lesions, comprising lipophilic extracts of juniper berries, *Juniperus comm. fruct.,* and at least one selected from a first group consisting of roots of *Symphytum off.,* flowers of *Calendula off.,* leaves of *Plantago lanceolata* and flowers of *Matricaria chamomilla,* in a fatty base.

2. The ointment for use according to claim 1, wherein the fatty base is comprised of >50% by weight of suet, and optionally additionally a plant oil.

3. The ointment for use according to claim 1 or 2, comprising at least two extracts selected from the first group.

4. The ointment for use according to claim 3, comprising at least three extracts selected from the first group.

5. The ointment for use according to claim 4, comprising all four extracts constituting the first group.

6. The ointment for use according to one of claims 1 to 5, further comprising at least one extract selected from a second group consisting of *Equiseti herba* and *Hypericum perforatum.*

7. The ointment for use according to claim 6, comprising all six extracts constituting the first and second groups.

8. A process of manufacturing the ointment for use according to one of claims 1 to 7, comprising heating the fatty base to above 100°C, admixing the ingredients, and filtering the mixture to obtain the filtrate as the ointment.

9. The process of claim 8, wherein all four ingredients of the first group are used, and the amounts of the dried ingredients are each at least one eighth and at most one half of the total amount of the ingredients.

10. The process of claim 8 or 9, wherein beeswax is added to the filtrate.

11. The process of claim 8, 9 or 10, wherein the total amount of the admixed dried ingredients is two thirds to one third of the mixture, and the amount of the fatty base is one third to two thirds of the mixture.

12. The process of one of claims 8 to 11, wherein the mixture is heated to at least 150°C.

13. The process of claim 12, wherein the mixture is kept at 150°C to 200°C for at least 1/2 hr.

14. The ointment for use according to one of claims 1 to 7, obtained from the process of one of claims 8 to 13.

15. A composition consisting of lipophilic extracts of juniper berries, *Juniperus comm. fruct.,* roots of *Symphytum off.,* flowers of *Calendula off,* leaves of *Plantago lanceolata* and flowers of *Matricaria chamomilla;* optionally beeswax, *Equiseti herba* and *Hypericum perforatum,* and suet as a fatty base, for use in the topical treatment of lesions of the skin.

## Patentansprüche

1. Salbe zur Anwendung in einem therapeutic Verfahren zur topischen Behandlung von Hautläsionen, die Salbe beinhaltend lipophile Extrakte von Wacholderbeeren, *Juniperus comm. fruct.,* und wenigstens eines ausgewählt au seiner ersten Gruppe, welche aus Wurzeln von *Symphytum off.,* Blüten von *Calendula off.,* Blättern von *Plantago lanceolata* und Blüten von *Matricaria chamomilla* besteht, in einer Fettbasis.

2. Salbe zur Anwendung gemäß Anspruch 1, wobei die Fettbasis aus >50% nach Gewicht Talg besteht, und optional zusätzlich einem Pflanzenöl.

3. Salbe zur Anwendung gemäß Anspruch 1 oder 2, beinhaltend wenigstens zwei der Extrakte der ersten Gruppe.

4. Salbe zur Anwendung gemäß Anspruch 3, beinhaltend wenigstens drei der Extrakte der ersten Gruppe.

5. Salbe zur Anwendung gemäß Anspruch 4, beinhaltend alle vier der Extrakte der ersten Gruppe.

6. Salbe zur Anwendung gemäß einem der Ansprüche 1 bis 5, ferner beinhaltend wenigstens einen Extrakt ausgewählt aus einer zweiten Gruppe, welche aus *Equiseti herba* und *Hypericum perforatum* besteht.

7. Salbe zur Anwendung gemäß Anspruch 6, beinhaltend alle sechs Extrakte der ersten und zweiten Gruppe.

8. Verfahren zur Herstellung der Salbe zur Anwendung gemäß einem der Ansprüche 1 bis 7, beinhaltend Erhitzen der Fettbasis auf über 100°C, Zumischen der Zutaten, und Filtern der Mischung, um das Filtrat als die Salbe zu erhalten.

9. Verfahren gemäß Anspruch 8, wobei alle vier Zutaten der ersten Gruppe verwendet werden, und die Anteile der getrockneten Zutaten jeweils mindestens ein Achtel und höchstens die Hälfte der Gesamtmenge der Zutaten betragen.

10. Verfahren gemäß Anspruch 8 oder 9, wobei dem Filtrat Bienenwachs zugegeben wird.

11. Verfahren gemäß Anspruch 8, 9 oder 10, wobei die Gesamtmenge der zugemischten getrockneten Zutaten zwei Drittel bis ein Drittel der Mischung beträgt, und die Menge der Fettbasis ein Drittel bis zwei Drittel der Mischung beträgt.

12. Verfahren gemäß einem der Ansprüche 8 bis 11, wobei die Mischung auf mindestens 150°C erhitzt wird.

13. Verfahren gemäß Anspruch 12, wobei die Mischung für mindestens ½ Stunde auf 150°C bis 200°C gehalten wird.

14. Salbe zur Anwendung gemäß einem der Ansprüche 1 bis 7, erhalten nach dem Verfahren gemäß einem der Ansprüche 8 bis 13.

15. Zusammensetzung bestehend aus lipophilen Extrakten von Wacholderbeeren, *Juniperus comm. fruct.,* Wurzeln von *Symphytum off.,* Blüten von *Calendula off,* Blättern von *Plantago lanceolata* und Blüten von *Matricaria chamomilla;* optional Bienenwachs, *Equiseti herba* und *Hypericum perforatum,* und Talg als Fettbasis, zur Anwendung bei der topischen Behandlung von Läsionen der Haut.

## Revendications

1. Pommade pour une utilisation dans un procédé thérapeutique de traitement topique de lésions cutanées, comprenant des extraits lipophiles de baies de genévrier, *Juniperus comm. fruct.,* et au moins un élément choisi dans un premier groupe constitué de racines de *Symphytum off.,* de fleurs de *Calendula off.,* de feuilles de *Plantago lanceolata* et de fleurs de *Matricaria chamomilla,* dans une base grasse.

2. Pommade pour une utilisation selon la revendication 1, dans laquelle la base grasse est composée de > 50 % en poids de suif, et optionnellement en outre d'une huile végétale.

3. Pommade pour une utilisation selon la revendication 1 ou 2, comprenant au moins deux extraits choisis dans le premier groupe.

4. Pommade pour une utilisation selon la revendication 3, comprenant au moins trois extraits choisis dans le premier groupe.

5. Pommade pour une utilisation selon la revendication 4, comprenant les quatre extraits constituant le premier groupe.

6. Pommade pour une utilisation selon l'une des revendications 1 à 5, comprenant en outre au moins un extrait choisi dans un second groupe constitué de *Equiseti herba* et *Hypericum perforatum.*

7. Pommade pour une utilisation selon la revendication 6, comprenant les six extraits constituant les premier et second groupes.

8. Procédé de fabrication de la pommade pour une utilisation selon l'une des revendications 1 à 7, comprenant le chauffage de la base grasse à plus de 100 °C, le mélange des composants, et la filtration du mélange pour obtenir le filtrat en tant que la pommade.

9. Procédé selon la revendication 8, dans lequel les quatre ingrédients du premier groupe sont utilisés, et les quantités des composants séchés sont chacun au moins d'un huitième et au plus d'une moitié de la quantité totale des composants.

10. Procédé selon la revendication 8 ou 9, dans lequel de la cire d'abeille est ajoutée au filtrat.

11. Procédé selon la revendication 8, 9 ou 10, dans lequel la quantité totale des composants séchés mélangés est de deux tiers à un tiers du mélange, et la quantité de la base grasse est d'un tiers à deux tiers du mélange.

12. Procédé selon l'une des revendications 8 à 11, dans lequel le mélange est chauffé à au moins 150 °C.

13. Procédé selon la revendication 12, dans lequel le mélange et maintenu à 150 °C à 200 °C pendant au moins 1/2 h.

14. Pommade pour une utilisation selon l'une des revendications 1 à 7, obtenue à partir du procédé selon l'une des revendications 8 à 13.

15. Composition constituée d'extraits lipophiles de baies de genévrier, *Juniperus comm. fruct.,* de racines de *Symphytum off.,* de fleurs de *Calendula off.,* de feuilles de *Plantago lanceolata* et de fleurs de *Matricaria chamomilla ;* optionnellement de cire d'abeille, de *Equiseti herba* et de *Hypericum perforatum,* et de suif en tant que base grasse, pour une utilisation dans le traitement topique de lésions de la peau.
